# EUROPEAN PATENT SPECIFICATION

(11) **EP 2 029 050 B1**
(45) Date of publication and mention of the grant of the patent: **17.08.2011**
(21) Application number: 07777236.6
(22) Date of filing: 23.05.2007
(51) Int. Cl.: A61F 2/04, A61F 5/00

(54) **ANTI-OBESITY FLOW CONTROLLER**
DURCHFLUSSREGLER GEGEN ADIPOSITAS
CONTRÔLEUR DE FLUX ANTI-OBÉSITÉ

(30) Priority: 30.05.2006 US 443544
(43) Date of publication of application: 04.03.2009
(73) Proprietor: Boston Scientific Limited, Christ Church (BB)
(72) Inventor: WEITZNER, Barry, Acton, MA 01720 (US)
(74) Representative: Vossius & Partner
(86) International application number: PCT/US2007/012264
(87) International publication number: WO 2007/142832

(56) References cited:
- EP-A- 1 618 855
- WO-A-2004/049982
- US-A- 5 820 584
- US-A1- 2003 040 804
- US-A1- 2005 125 075
- US-A1- 2006 184 194

## Description

### Cross-Reference to Related Application

This international application claims priority to U.S. Patent Application No. 11/443,544, filed May 30, 2006

### Field of the Invention

The present invention relates generally to anti-obesity flow controllers and methods for using the same. More specifically, the present invention relates to anti-obesity flow controllers which are secured in the duodenum adjacent to the stomach to reduce digestion and absorption of food.

### Background of the Invention

The incidence of obesity and its associated health-related problems has become significant. The causes of obesity involve a complex interplay of genetic, environmental, psycho-behavioral, endocrine, metabolic, cultural, and socio-economic factors. Severe obesity is frequently associated with significant comorbid medical conditions, including coronary artery disease, hypertension, type II diabetes mellitus, gallstones, nonalcoholic steatohepatitis, pulmonary hypertension, and sleep apnea. Obesity is a leading cause of preventable death in the U.S. The spectrum of comorbid conditions associated with obesity includes cancer, osteoarthritis, and heart disease. The economic cost of obesity is substantial.

Current treatments for obesity range from diet, exercise, behavioral modification, and pharmacotherapy to various types of surgery, with varying risks and efficacy. In general, nonsurgical treatments, although less invasive, achieve only relatively short-term and limited weight loss in most patients. Non-surgical treatments are utilized for patients such as with a body-mass index (BMI) which is greater than 30, and have not proven very effective. Surgical treatments include gastroplasty to restrict the capacity of the stomach to hold large amounts of food, such as by stapling or "gastric banding". Other surgical procedures include gastric bypass and gastric "balloons" which, when deflated, may be inserted into the stomach and then are distended by filling with saline solution.

Surgical interventions may be performed on those patients with a BMI which is greater than 40 (deemed morbidly obese). Surgical interventions may include restrictive operations that reduce the size of the stomach pouch to limit food intake. Surgical interventions may also include malabsorbative procedures that rearrange the small intestine in an attempt to decrease the functional length or efficiency of nutrient absorption, or a combination thereof. One combination procedure is Gastric Bypass (GPB or Roux-en-Y) which has been effective for most patients who maintain about 70% of excess weight loss after 5 years, and 50% thereof after 10 years. Both of these types of procedures may be performed Iaparoscopically, but may have complications. Also, GPB is normally irreversible. Other treatment approaches are being considered. U.S. Publication No. 2003/0040804 A1 discloses a satiation device. Excess weight loss is the loss of weight which is greater than the ideal body weight.

The need exists for low cost, less invasive interventions for the treatment of obesity, including morbid obesity.

### Summary of the Invention

The anti-obesity flow controller of the present invention includes a tubular structure having outer and inner surfaces, and proximal and distal ends. The tubular structure has a lumen which has an outer periphery defined by the inner surface. The tubular structure is sized to fit within a duodenum in substantially coaxial relation therewith. The tubular structure is impervious or semi-permeable to digestive fluid and chyme within the duodenum. Chyme is the partially digested food which flows into the duodenum from the stomach. The anti-obesity flow controller includes a transport structure at least a part of which is connected to the outer surface. The transport structure extends to the distal end of the tubular structure.

A retainer structure is connected to the tubular structure. The retainer structure secures the tubular structure within the duodenum such that the transport structure is positioned to receive the digestive fluid from a papilla of Vater on an inner surface of the duodenum. The transport structure provides a conduit for the digestive fluid therein to flow to the distal end. The retainer structure further secures the tubular structure within the duodenum such that the proximal end has a proximal position relative to the papilla of Vater. The retainer structure further secures the tubular structure within the duodenum such that the distal end has a distal position.relative to the papilla of Vater. The retainer structure further secures the tubular structure within the duodenum such that the proximal end communicates with the pylorus to provide for the chyme therefrom to flow into the lumen. A controller structure is connected to the inner surface of the tubular structure to change the velocity of the chyme which flows within the lumen from the proximal to distal ends.

The anti-obesity flow controller, when secured in the proper location within the duodenum, reduces or prevents mixing of the chyme and digestive fluid within the duodenum. The digestive fluid within the duodenum includes biliary and pancreatic juices which reach the interior of the duodenum by flowing through the papilla of Vater which is contiguous with the inner surface of the duodenum. The digestive fluid is supplied to the papilla of Vater by the bile and pancreatic ducts. The anti-obesity flow controller reduces or prevents mixing of the chyme and digestive fluid by reducing or preventing the digestive fluid which flows through the papilla of Vater from passing through the tubular structure. Consequently, mixing of the digestive fluid with the chyme in the region of the duodenum which is occupied by the anti-obesity flow controller is reduced or prevented. This reduces the exposure of the chyme to the digestive fluid which reduces the associated chemical breakdown thereof. This is a result of the tubular structure being semi-permeable or impervious to the chyme. The reduction in the mixing of the chyme and digestive fluid provided by the anti-obesity flow controller reduces the caloric intake by the patient. Also, this reduction in the mixing reduces the breakdown of fats because the bile is separated from the chyme over the axial length of the anti-obesity flow controller. Consequently, the chemical transformation of the chyme by the digestive fluid which is normally required for absorption of the nutrients, fats and other substances in the chyme by the duodenum is reduced.

Additionally, the anti-obesity flow controller reduces the absorption of the nutrients, fats and other substances in the chyme by the duodenum. This reduced absorption results from the tubular structure being semi-permeable or impervious to the chyme. As a result, the chyme which is contained within the tubular structure is partially or completely prevented from reaching the inner surface of the portion of the duodenum in which the anti-obesity flow controller is located. Consequently, the portion of the duodenum in which the anti-obesity flow controller is located is partially or completely prevented from absorbing the nutrients, fats and other substances in the chyme. Reducing the absorption of the nutrients, fats and other substances by the duodenum reduces the caloric intake by the patient. Also, reducing the absorption of the nutrients, fats and other substances reduces the fat intake by the patient which typically reduces the weight thereof.

The anti-obesity flow controller separates the food and chyme, which flows from the stomach into the duodenum, from the digestive fluid which includes bile acids and pancreatic enzymes and which promotes lipid absorption. This separation by the anti-obesity flow controller is provided at the location thereof in the duodenum which is the beginning of the small intestine. The anti-obesity flow controller treats obesity using a mal-absorptive method. Separating the food from the digestive fluid may reduce the amount of digestion and, consequently, the amount of weight a person gains from eating a specific quantity of food.

The anti-obesity flow controller reduces or prevents mixing of the chyme and digestive fluid by changing the velocity of the chyme which flows within the lumen. This causes the chyme and digestive fluid to reach the distal end of the tubular structure at different times since the digestive fluid normally enters the duodenum through the papilla of Vater at generally the time during which the chyme flows past the papilla of Vater. Consequently, the mixing of the chyme and digestive fluid is prevented or reduced since either the chyme or digestive fluid is downstream of the distal end when the other arrives thereat, depending upon whether the velocity of the chyme is increased or decreased. For example, increasing the velocity of the chyme results in the chyme being downstream of the distal end when the digestive fluid arrives thereat. Alternatively, decreasing the velocity of the chyme results in the digestive fluid being downstream of the distal end when the chyme arrives thereat. The longitudinal gap between the chyme and digestive fluid during the respective arrivals thereof at the distal end reduces or prevents mixing thereof downstream of the distal end within the duodenum.

These and other features of the invention will be more fully understood from the following description of specific embodiments of the invention taken together with the accompanying drawings.

### Brief Description of the Drawings

In the drawings:

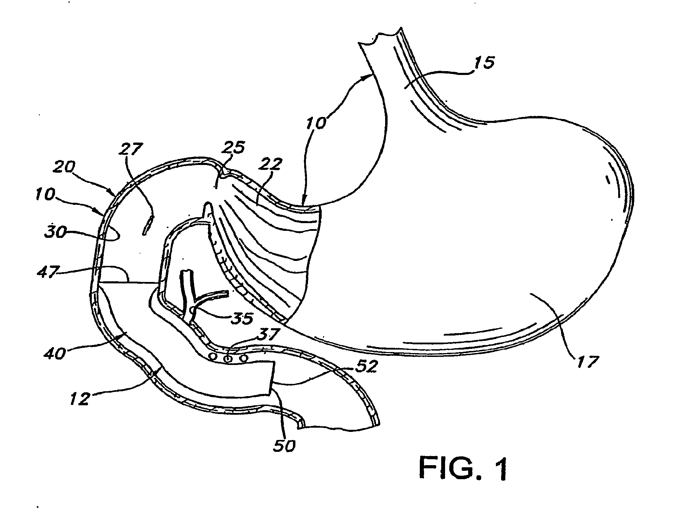

Fig. 1 is an anatomical elevational view of a stomach, duodenum and adjacent portions of the alimentary canal, the wall of the pyloric portion of the stomach and duodenum being broken away to show an anti-obesity flow controller in accordance with the present invention;

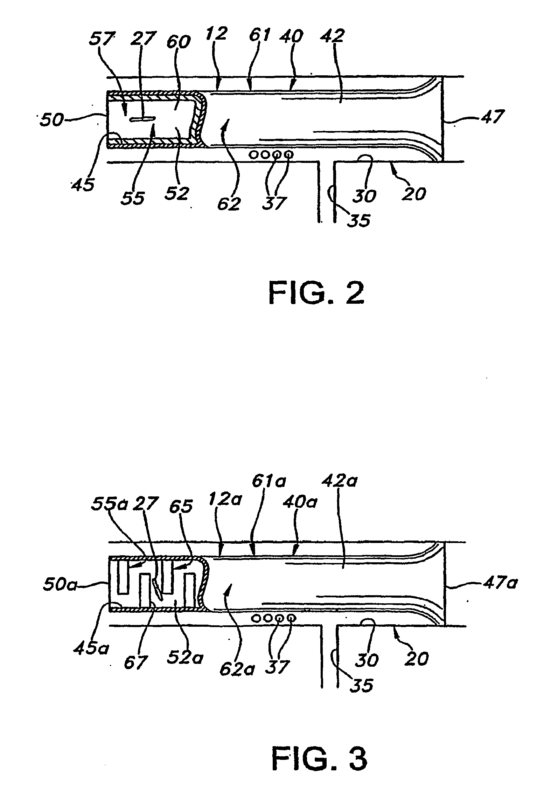

Fig. 2 is a side elevational view of the anti-obesity flow controller of Fig. 1, the anti-obesity flow controller being shown as having a tubular structure, the tubular structure and duodenum being illustrated as having substantially straight configurations, the tubular structure being shown as having a section broken away to show the controller structure including a liner structure; and
Fig. 3 is a side elevational view of an alternative embodiment of the anti-obesity flow controller of Fig. 2, the anti-obesity flow controller being shown as having a stent, the stent and duodenum being illustrated as having substantially straight configurations, the stent being shown as having a section broken away to show the controller structure including a restriction structure.

Corresponding reference characters indicate corresponding parts throughout the several views of the drawings.

### Detailed Description of the Invention

Referring to the drawings and more particularly to Fig. 1, a central portion of the alimentary canal 10 in which the anti-obesity flow controller 12 is located is illustrated. This portion of the alimentary canal 10 includes the distal segment of the esophagus 15, the stomach 17, and the duodenum 20. The duodenum 20 is the proximate segment of the small intestine. The stomach 17 has a pyloric portion 22 which leads to the duodenum 20 by way of the gastric outlet or pylorus 25. The pylorus 25 forms the distal aperture of the stomach and has an enclosing circular layer of muscle which is normally contracted to close the aperture but which relaxes to provide an open but restrictive passage. Although subject to substantial variation in different individuals, the pylorus 25 has a maximum open diameter of about 2 cm and the duodenum 20 has a diameter which typically is about 18 to 20 mm in a representative patient. The chyme 27 passes from the pyloric portion 22 through the pylorus 25 into the duodenum 20. The duodenum 20 has an inner surface 30 and a papilla of Vater 32 which is a trumpet-mouthed dilatation of the duodenal wall at the opening of the fused bile and pancreatic ducts. The digestive fluid 37 is supplied through the papilla of Vater 35, and flows into the interior of the duodenum 20.

The anti-obesity flow controller 12 is located within the duodenum 20 as shown in Fig. 1. The anti-obesity flow controller 12 includes a tubular structure which is defined by a tubular stent 40 having outer and inner surfaces 42, 45. The stent 40 has proximal and distal ends 47, 50. The stent 40 has a lumen 52 the outer periphery of which is defined by the inner surface 45.

The stent 40 may be formed of expanded polytetrafluoroethylene (ePTFE) or polyurethane. The stent 40 may be formed of biocompatible materials, such as polymers which may include fillers such as metals, carbon fibers, glass fibers or ceramics. Such polymers may include olefin polymers, polyethylene, polypropylene, polyvinyl chloride, polytetrafluoroethylene which is not expanded, fluorinated ethylene propylene copolymer, polyvinyl acetate, polystyrene, poly(ethylene terephthalate), naphthalene dicarboxylate derivatives, such as polyethylene naphthalate, polybutylene naphthalate, polytrimethylene naphthalate and trimethylenediol naphthalate, polyurethane, polyurea, silicone rubbers, polyamides, polycarbonates, polyaldehydes, natural rubbers, polyester copolymers, styrenebutadiene copolymers, polyethers, such as fully or partially halogenated polyethers, copolymers, and combinations thereof. Also, polyesters, including polyethylene terephthalate (PET) polyesters, polypropylenes, polyethylenes, polyurethanes, polyolefins, polyvinyls, polymethylacetates, polyamides, naphthalane dicarboxylene derivatives, and natural silk may be included in the stent 40.

The stent 40 may be formed of materials such as nitinol, Elgiloy, stainless steel, cobalt chromium, including MP35N, cobalt-based alloy, tantalum, niobium, platinum, gold, titanium, combinations thereof and other biocompatible metals, polymers and materials. Additionally, the stent 40 may include structural members which have an inner core formed of tantalum, gold, platinum, iridium, or a combination thereof, and an outer cladding of nitinol to provide composite members for improved radio-opacity or visibility. Examples of such composite members are disclosed in U.S. Patent Application Publication No. 2002/0035396 which is hereby incorporated by reference herein.

The stent 40 may be a WALLSTENT® RX Biliary Endoprosthesis made by the Boston Scientific Corporation. Alternatively, the stent 40 may be a NIR® Biliary Stent System made by the Boston Scientific Corporation.

The stent 40 may have various embodiments. For example, the stent 40 may be self-expanding or expandable by a balloon. The stent 40 may include one or more coiled stainless steel springs, helically wound coil springs including a heat-sensitive material, or expanding stainless steel stents formed of stainless steel wire in a zig-zag pattern. The stent 40 may be capable of radially contracting or expanding, such as by radial or circumferential distension or deformation. Self-expanding stents include stents which mechanically urge the stent to radially expand, and stents which expand at one or more specific temperatures as a result of the memory properties of the stent material for a specific configuration. Nitinol is a material which may be included in the stent 40 for providing radial expansion thereof both by mechanical urging, or by the memory properties of the nitinol based on one or more specific temperatures. The stent 40 may include one or more of the stents disclosed in U.S. Patent Nos. 4,503,569, 4,733,665, 4,856,516, 4,580,568, 4,732,152, and 4,886,062,

The stent 40 may be covered by a tubular cover structure which may be formed of various materials. For example, the cover structure may be a PERMALUME® covering for a Stent constituted by the WALLSTENT® RX Biliary Endoprosthesis which are made by the Boston Scientific Corporation. Alternatively, the cover structure may be formed of ePTFE.

The controller structure 55 includes a tubular liner structure 57 which is secured to the inner surface 45 of the stent 40 in coaxial relation therewith. The liner structure 57 has an inner surface 60. The liner structure 57 includes a material which provides for the inner surface 60 to have a material which is lubricious. In one embodiment of the liner structure 57, the material included therein provides for the inner surface 60 to have ePTFE, an embodiment of which is Teflon^{®}. In a further alternative embodiment of the liner structure 57, the material included therein provides for the material of the inner surface 60 to be hydrophilic.

The stent 40 is sized to fit within the duodenum 20 in substantially coaxial relation therewith. The anti-obesity flow controller 12 has a transport structure 61 at least a part of which is connected to the outer surface 42 of the stent 40. The transport structure 61 extends to the distal end 50.

The anti-obesity flow controller 12 includes a retainer structure 62 which is connected to the outer surface 42 of the stent 40. The retainer structure 62 secures the stent 40 within the duodenum 20 such that the transport structure 61 is positioned to receive the digestive fluid 37 from the papilla of Vater 35. The transport structure 61 provides a conduit for the digestive fluid 37 therein to flow to the distal end 50. The retainer structure 62 further secures the stent 40 within the duodenum 20 such that the proximal end 47 has a proximal position relative to the papilla of Vater 35. The retainer structure 62 further secures the stent 40 within the duodenum 20 such that the distal end 50 has a distal position relative to the papilla of Vater.35. The retainer structure 62 further secures the stent 40 within the duodenum 20 such that the proximal end 47 communicates with the pylorus 25. This provides for the chyme 27 from the pylorus 25 to flow into the lumen 52.

Either or both of the stent 40 and liner structure 57 are impervious or semi-permeable to the chyme 27 which is within the lumen 52 which is located within the duodenum 20. Consequently, the chyme 27 is completely or partially prevented from passing in the transverse direction through the liner structure 57 and stent 40 to reach the outer surface 42. Also, either or both of the stent 40 and liner structure 57 are impervious or semi-permeable to the digestive fluid 37. Consequently, the digestive fluid 37 which is within the duodenum 20 between the inner surface 30 thereof and the outer surface 42 is completely or partially prevented from passing in the transverse direction through the stent 40 and liner structure 57 to reach the inner surface 60.

In an alternative embodiment, a tubular cover structure may be secured to the outer surface 42 to completely or partially prevent passage in the transverse direction of the chyme 27 and digestive fluid 37 between the lumen 52 and region between the inner surface 30 and outer surface of the cover structure. Such a cover structure may be impervious or semi-permeable to the chyme 27 and digestive fluid 37 such that neither the stent 40 nor the liner structure 57 are required to be impervious or semi-permeable to the chyme and digestive fluid. Alternatively, such a cover structure may be impervious or semi-permeable to the chyme 27 and digestive fluid 37 in addition to either or both of the stent 40 and liner structure 57 being impervious or semi-permeable to the chyme and digestive fluid.

The chyme 27 within the lumen 52 flows from the proximal to distal ends 47, 50. The inner surface 60 of the liner structure 57 includes one or more materials which, when contacted by the chyme 27, causes an increase in the velocity of the chyme which flows within the lumen 52 from the proximal to distal ends 47, 50. Such an increase in the velocity of the chyme 27 may be provided by the inner surface 60 having one or more materials which are lubricious, such as ePTFE or a material which is hydrophilic.

The contact between the chyme 27 and inner surface 60 reduces the duration of the flow of the chyme through the lumen 52 from the proximal to distal ends 47, 50. Consequently, the chyme 27 within the lumen 52 typically exits therefrom at the distal end 50 before the exit of the digestive fluid 37 from the region between the outer surface 42 and inner surface 30 of the duodenum 20 at the distal end 50. The exit of the chyme 27 before the exit of the digestive fluid 37 at the distal end 50 results from the flow of the chyme into the duodenum 20 and the substantially simultaneous supply of the digestive fluid into the interior thereof, and the reduced duration of the flow of the chyme through the lumen 52 relative to the duration of the flow of the digestive fluid through the region between the outer surface 42 and inner surface 30 of the duodenum. The exit of the chyme 27 before the exit of the digestive fluid 37 at the distal end 50 reduces the mixing of the digestive fluid and chyme since substantially all or at least a portion of the chyme is downstream of the distal fluid during the exit thereof at the distal end within the duodenum 20. The digestive fluid 37 which enters the duodenum 20 without mixing with the chyme 27 may be absorbed by the inner surface 30 of the duodenum 20. This reduces the mixing of the digestive fluid 37 and chyme 27 which reduces the digestion thereof and absorption of the nutrients, fats, and other substances in the chyme by the inner surface 30.

The stent 40 and controller structure 55 may be treated with anti-thrombogenic agents (such as heparin, heparin derivatives, urokinase, and PPack (dextrophenylalanine proline arginine chloromethylketone)), anti-proliferative agents (such as enoxaprin, angiopeptin, or monoclonal antibodies capable of blocking smooth muscle cell proliferation, hirudin, and acetylsalicylic acid), anti-inflammatory agents (such as dexamethasone, prednisolone, corticosterone, budesonide, estrogen, sulfasalazine, and mesalamine), antineoplastic/antiproliferative/anti-miotic agents (such as paclitaxel, 5-fluorouracil, cisplatin, vinblastine, vincristine, epothilones, endostatin, angiostatin and thymidine kinase inhibitors), anesthetic agents (such as lidocaine, bupivacaine, and ropivacaine), anti-coagulants (such as D-Phe-Pro-Arg chloromethyl keton, an RGD peptide-containing compound, heparin, antithrombin compounds, platelet receptor antagonists, anti-thrombin antibodies, anti-platelet receptor antibodies, aspirin, prostaglandin inhibitors, platelet inhibitors and tick antiplatelet peptides), vascular cell growth promotors (such as growth factor inhibitors, growth factor receptor antagonists, transcriptional activators, and translational promotors), vascular cell growth inhibitors (such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin), cholesterol-lowering agents, vasodilating agents, and agents which interfere with endogenous vascoactive mechanisms.

An alternative embodiment of the anti-obesity flow controller 12a is shown in Fig. 3. Parts illustrated in Fig. 3 which correspond to parts illustrated in Figs. 1 and 2 have, in Fig. 3, the same reference numeral as in Figs. 1 and 2 with the addition of the suffix "a". In this alternative embodiment, the anti-obesity flow controller 12a includes a controller structure 55a which has a restriction structure 65. The restriction structure 65 includes a baffle structure 67.

Either or both of the stent 40a and baffle structure 67 may be impervious or semi-permeable to the chyme 27 and digestive fluid 37. In an alternative embodiment, the baffle structure 67 may be secured to sections of the inner surface 45a and the stent 40 may have transverse apertures, such as in a stent including elongate structural members such as wires. In such an alternative embodiment, a tubular cover structure may be secured to the outer surface 42a to completely or partially prevent passage in the transverse direction of the chyme 27 and digestive fluid 37 between the lumen 52a and region between the inner surface 30 and outer surface of the cover structure. Such a cover structure would be impervious or semi-permeable to the chyme 27 and digestive fluid 37. In a further alternative embodiment, such a cover structure may be impervious or semi-permeable to the chyme 27 and digestive fluid 37 in addition to either or both of the stent 40a and baffle structure 67 being impervious or semi-permeable to the chyme and digestive fluid.

The baffle structure 65 requires the chyme 27 which flows through the lumen 52a from the proximal to distal ends 47a, 50a to follow a circuitous or serpentine path through the lumen. This circuitous or serpentine path provides for a decrease in the velocity of the chyme 27 which flows through the lumen 52a from the proximal to distal ends 47a, 50a.

The decrease in the velocity of the chyme 27 which is within the lumen 52a increases the duration of the flow of the chyme through the lumen from the proximal to distal ends 47a, 50a. Consequently, the chyme 27 within the lumen 52a typically exits therefrom at the distal end 50a after the exit of the digestive fluid 37 from the region between the outer surface 42a and inner surface 30 of the duodenum 20 at the distal end 50a. The delay in the exit of the chyme 27 through the distal end 50a results from the flow of the chyme into the duodenum 20 and the substantially simultaneous supply of the digestive fluid 37 to the region between the outer surface 42a and inner surface 30, and the increased duration of the flow of the chyme through the lumen 52a relative to the duration of the flow of the digestive fluid 37 through the region between the outer surface 42a and inner surface 30. The delayed exit of the chyme 27 through the distal end 50a relative to the exit of the digestive fluid 37 from the region between the outer surface 42a and inner surface 30 at the distal end 50a reduces the mixing of the digestive fluid and chyme since substantially all or at least a portion of the digestive fluid is downstream of the chyme during the exit thereof at the distal end within the duodenum 20. The digestive fluid 37 which enters the duodenum 20 without mixing with the chyme 27 may be absorbed by the inner surface 30. This reduces the mixing of the digestive fluid 37 and chyme 27 which reduces the digestion thereof and absorption of the chyme and associated nutrients by the inner surface 30. Also, the exit of the digestive fluid 37 at the distal end 50a before the exit of the chyme thereat reduces the breakdown of the fats in the chyme which reduces the absorption of the fats by the inner surface 30.

The contact of the chyme 27 which flows within the lumens 52, 52a from the proximal to distal ends 47, 47a, 50, 50a with the controller structures 55, 55a provides changes in the velocity of the chyme. More specifically, the contact between the chyme 27 and controller structure 55 results in an increase in the velocity of the chyme. Alternatively, the contact between the chyme 27 and controller structure 55a results in a decrease in the velocity of the chyme. Alternative embodiments of the controller structures 55, 55a are possible which are integral with the stent 40 or alternative embodiments thereof which provide the tubular structure.

An anti-obesity flow controller, such as the anti-obesity flow controller 12, may be used according to a method for inducing weight loss in a patient. The method not claimed by the invention includes inserting a tubular structure of the anti-obesity flow controller into a duodenum, such as the duodenum 20, in substantially coaxial relation therewith. An embodiment of the tubular structure to which this inserting may be applied is the stent 40. The tubular structure has outer and inner surfaces, proximal and distal ends, and a lumen. The anti-obesity flow controller has a transport structure at least a part of which is connected to the outer surface of the tubular structure. The transport structure extends to the distal end. The anti-obesity flow controller includes a controller structure which is connected to the inner surface of the tubular structure.

The method further includes locating the tubular structure within and longitudinally relative to the duodenum such that the transport structure is positioned to receive a digestive fluid, such as the digestive fluid 37, from a papilla of Vater, such as the papilla of Vater 35. An embodiment of the transport structure which may be positioned according to this locating includes the transport structure 61.

The locating further positions the anti-obesity flow controller such that the proximal end of the tubular structure has a proximal position relative to the papilla of Vater. An embodiment of the proximal end of the tubular structure which may be positioned according to this locating includes the proximal end 47.

The locating further positions the anti-obesity flow controller such that the distal end of the tubular structure has a distal position relative to the papilla of Vater. An embodiment of the distal end of the tubular structure which may be positioned according to this locating includes the distal end 50.

The locating further positions the anti-obesity flow controller such that the lumen of the tubular structure receives a chyme, such as the chyme 27, from a pylorus, such as the pylorus 25. An embodiment of the lumen of the tubular structure which may be positioned according to this locating includes the lumen 52.

The method further includes engaging a retainer structure of the anti-obesity flow controller with the inner surface of the duodenum, such as the inner surface 30. The engaging secures the transport structure in the position thereof to receive the digestive fluid from the papilla of Vater.

The engaging further secures the lumen in the position thereof to receive the chyme from the pylorus such that the controller structure changes the velocity of the chyme which flows within the lumen of the tubular structure from the proximal to distal ends thereof. An embodiment of the controller structure to which this engaging may be applied is the controller structure 55.

U.S. Patent No. 6,740,121 is hereby referenced. The following U.S. Patent Applications are hereby referenced

Title: Anti-Obesity Stent; Inventors: Barry Weitzner, Taryn Deneault, Katie Krueger, Claude Clerc, Harold W. Martins, and William Bertolino; Filed on same date as present U.S. Patent Application; Attorney Docket No.: 792-27;

Title: Anti-Obesity Dual Stent; Inventors: Katie Krueger, William Bertolino, Barry Weitzner, and Claude Clerc; Filed on same date as present U.S. Patent Application; Attorney Docket No.: 792-40; and

Title: Anti-Obesity Diverter Structure; Inventors: Katie Krueger, and Harold W. Martins; Filed on same date as present U.S. Patent Application; Attorney Docket No.: 792-42.

While the invention has been described by reference to certain preferred embodiments, it should be understood that numerous changes could be made within the scope of the inventive concept described. Accordingly, it is intended that the invention not be limited to the disclosed embodiments, but that it have the full scope permitted by the language of the following claims.

## Claims

1. An anti-obesity flow controller (12,12a) comprising:
a tubular structure (40) having outer and inner surfaces (42,45), said tubular structure (40) having proximal and distal ends (47,50), said tubular structure (40) having a lumen (52) which has an outer periphery defined by said inner surface (45), said tubular structure (40) being sized to fit within a duodenum in substantially coaxial relation therewith, said tubular structure (40) being impervious or semi-permeable to a digestive fluid and chyme within the duodenum; and **characterized by**:
a transport structure (61) at least a part of which is connected to said outer surface (42), said transport structure (61)extending to said distal end (50);
a retainer structure (62) connected to said tubular structure (40), said retainer structure (62) securing said tubular structure (40) within the duodenum such that said transport structure (61) is positioned to receive the digestive fluid from a papilla of Vater on an inner surface of the duodenum, said transport structure (61) providing a conduit for the digestive fluid therein to flow to said distal end, said retainer structure (62) further securing said tubular structure (40) within the duodenum such that said proximal end has a proximal position relative to the papilla of Vater, said retainer structure (62) further securing said tubular structure (40) within the duodenum such that said distal end has a distal position relative to the papilla of Vater, said retainer structure (62) further securing said tubular structure (40) within the duodenum such that said proximal end (47) communicates with the pylorus to provide for the chyme therefrom to flow into said lumen (52); and
a controller structure (55) connected to said inner surface (45) of said tubular structure (40) to change a velocity of the chyme which flows within said lumen (52) from said proximal to distal ends; wherein the tubular structure (40) extends along the length of the controller structure (55).

2. An anti-obesity flow controller according to claim 1, wherein said controller structure (55) comprises a liner structure (57) secured to said inner surface (45) of said tubular structure (40),
said liner structure (57) having an inner surface (60) which, when contacted by the chyme which flows within said lumen (52), results in an increase in the velocity of the chyme.

3. An anti-obesity flow controller according to claim 2, wherein said tubular structure (40) comprises a tubular stent having an inner surface,
said liner structure (57) being tubular and secured to said inner surface of said stent in coaxial relation therewith.

4. An anti-obesity flow controller according to claim 2, wherein said inner surface (60) of said liner structure (57) comprises a material which is lubricious such that, when said material is contacted by the chyme which flows within said lumen, the velocity of the chyme increases.

5. An anti-obesity flow controller according to claim 2, wherein said inner surface (60) of said liner structure (57) comprises ePTFE which, when contacted by the chyme which flows within said lumen, results in an increase in the velocity of the chyme.

6. An anti-obesity flow controller according to claim 2, wherein said inner surface (60) of said liner structure (57) comprises a material which is hydrophilic such that, when said material is contacted by the chyme which flows within said lumen, the velocity of the chyme increases.

7. An anti-obesity flow controller according to claim 1, wherein said controller structure (55a) comprises a restriction structure (65) secured to said inner surface (45a) of said tubular structure (40a), said restriction structure (65) providing an obstruction to the chyme which flows within said lumen (52a) resulting in a decrease in the velocity of the chyme.

8. An anti-obesity flow controller according to claim 7, wherein said restriction structure (65) comprises a baffle structure (67) secured to said inner surface (45a) of said tubular structure (40a), said baffle structure (67) providing an obstruction to the chyme which flows within said lumen (52a) resulting in a decrease in the velocity of the chyme.

## Patentansprüche

1. Anti-Adipositas-Durchflussregler (12, 12a), der aufweist:
eine Röhrenstruktur (40) mit einer Außen- und einer Innenfläche (42, 45), wobei die Röhrenstruktur (40) ein proximales und ein distales Ende (47, 50) hat, die Röhrenstruktur (40) ein Lumen (52) hat, das einen durch die Innenfläche (45) festgelegten Außenumfang hat, die Röhrenstruktur (40) so bemessen ist, dass sie sich in ein Duodenum im Wesentlichen in koaxialer Beziehung dazu einpasst und die Röhrenstruktur (40) für einen Verdauungssaft und Chymus im Duodenum undurchlässig oder semipermeabel ist; und **gekennzeichnet durch**:
eine Transportstruktur (61), von der mindestens ein Teil mit der Außenfläche (42) verbunden ist, wobei sich die Transportstruktur (61) zum distalen Ende (50) erstreckt;
eine Retentionsstruktur (62), die mit der Röhrenstruktur (40) verbunden ist, wobei die Retentionsstruktur (62) die Röhrenstruktur (40) im Duodenum so befestigt, dass die Transportstruktur (61) so positioniert ist, dass sie den Verdauungssaft aus einer Vater-Papille auf einer Innenfläche des Duodenums aufnimmt, wobei die Transportstruktur (61) einen Kanal für den Verdauungssaft darin bildet, damit er zum distalen Ende fließt, wobei die Retentionsstruktur (62) die Röhrenstruktur (40) im Duodenum ferner so befestigt, dass das proximale Ende eine proximale Position relativ zur Vater-Papille hat, wobei die Retentionsstruktur (62) die Röhrenstruktur (40) im Duodenum ferner so befestigt, dass das distale Ende eine distale Position relativ zur Vater-Papille hat, und wobei die Retentionsstruktur (62) die Röhrenstruktur (40) im Duodenum ferner so befestigt, dass das proximale Ende (47) mit dem Pylorus kommuniziert, um dafür zu sorgen, dass der Chymus aus ihm in das Lumen (52) fließt; und
eine Reglerstruktur (55), die mit der Innenfläche (45) der Röhrenstruktur (40) verbunden ist, um eine Geschwindigkeit des Chymus zu ändern, der im Lumen (52) vom proximalen zum distalen Ende fließt; wobei sich die Röhrenstruktur (40) über die Länge der Reglerstruktur (55) erstreckt.

2. Anti-Adipositas-Durchflussregler nach Anspruch 1, wobei die Reglerstruktur (55) eine Auskleidungsstruktur (57) aufweist, die an der Innenfläche (45) der Röhrenstruktur (40) befestigt ist,
wobei die Auskleidungsstruktur (57) eine Innenfläche (60) hat, die bei Kontakt mit dem Chymus, der in Lumen (52) fließt, zu einer Zunahme der Geschwindigkeit des Chymus führt.

3. Anti-Adipositas-Durchflussregler nach Anspruch 2, wobei die Röhrenstruktur (40) einen röhrenförmigen Stent mit einer Innenfläche aufweist,
wobei die Auskleidungsstruktur (57) röhrenförmig und an der Innenfläche des Stents in koaxialer Beziehung dazu befestigt ist.

4. Anti-Adipositas-Durchflussregler nach Anspruch 2, wobei die Innenfläche (60) der Auskleidungsstruktur (57) ein Material aufweist, das gleitfähig ist, so dass bei Kontakt des Materials mit dem Chymus, der im Lumen fließt, die Geschwindigkeit des Chymus zunimmt.

5. Anti-Adipositas-Durchflussregler nach Anspruch 2, wobei die Innenfläche (60) der Auskleidungsstruktur (57) ePTFE aufweist, das bei Kontakt mit dem Chymus, der im Lumen fließt, zu einer Zunahme der Geschwindigkeit des Chymus führt.

6. Anti-Adipositas-Durchflussregler nach Anspruch 2, wobei die Innenfläche (60) der Auskleidungsstruktur (57) ein Material aufweist, das hydrophil ist, so dass bei Kontakt des Materials mit dem Chymus, der im Lumen fließt, die Geschwindigkeit des Chymus zunimmt.

7. Anti-Adipositas-Durchflussregler nach Anspruch 1, wobei die Reglerstruktur (55a) eine Restriktionsstruktur (65) aufweist, die an der Innenfläche (45a) der Röhrenstruktur (40a) befestigt ist, wobei die Restriktionsstruktur (65) für eine Obstruktion für den Chymus sorgt, der im Lumen (52a) fließt, was zu einer Abnahme der Geschwindigkeit des Chymus führt.

8. Anti-Adipositas-Durchflussregler nach Anspruch 7, wobei die Restriktionsstruktur (65) eine Umlenkstruktur (67) aufweist, die an der Innenfläche (45a) der Röhrenstruktur (40a) befestigt ist, wobei die Umlenkstruktur (67) für eine Obstruktion für den Chymus sorgt, der im Lumen (52a) fließt, was zu einer Abnahme der Geschwindigkeit des Chymus führt.

## Revendications

1. Contrôleur de flux anti-obésité (12, 12a) comprenant :
une structure tubulaire (40) ayant des surfaces externe et interne (42, 45), ladite structure tubulaire (40) ayant des extrémités proximale et distale (47, 50), ladite structure tubulaire (40) ayant une lumière (52) qui présente une périphérie externe définie par ladite surface interne (45), ladite structure tubulaire (40) étant dimensionnée pour s'adapter à l'intérieur d'un duodénum en relation sensiblement coaxiale avec lui, ladite structure tubulaire (40) étant imperméable ou semi-perméable à un fluide digestif et un chyme à l'intérieur du duodénum ; et **caractérisé par** :
une structure de transport (61) dont au moins une partie est reliée à ladite surface externe (42), ladite structure de transport (61) s'étendant vers ladite extrémité distale (50);
une structure de retenue (62) reliée à ladite structure tubulaire (40), ladite structure de retenue (62) fixant ladite structure tubulaire (40) à l'intérieur du duodénum de telle sorte que ladite structure de transport (61) soit positionnée pour recevoir le fluide digestif à partir d'une papille de Vater sur une surface interne du duodénum,
ladite structure de transport (61) procurant un conduit pour que le fluide digestif à l'intérieur s'écoule vers ladite extrémité distale, ladite structure de retenue (62) fixant en outre ladite structure tubulaire (40) à l'intérieur du duodénum de telle sorte que ladite extrémité proximale ait une position proximale par rapport à la papille de Vater,
ladite structure de retenue (62) fixant en outre ladite structure tubulaire (40) à l'intérieur du duodénum de telle sorte que ladite extrémité distale ait une position distale par rapport à la papille de Vater, ladite structure de retenue (62) fixant en outre ladite structure tubulaire (40) à l'intérieur du duodénum de telle sorte que ladite extrémité proximale (47) communique avec le pylore pour permettre au chyme provenant de celui-ci de s'écouler dans ladite lumière (52) ; et
une structure de contrôle (55) reliée à ladite surface interne (45) de ladite structure tubulaire (40) pour modifier une vitesse du chyme qui s'écoule à l'intérieur de ladite lumière (52) depuis lesdites extrémités proximale à distale, la structure tubulaire (40) s'étendant le long de la structure de contrôle (55).

2. Contrôleur de flux anti-obésité selon la revendication 1, dans lequel ladite structure de contrôle (55) comprend une structure de revêtement (57) fixée à ladite surface interne (45) de ladite structure tubulaire (40),
ladite structure de revêtement (57) ayant une surface interne (60) qui, lorsque le chyme s'écoulant à l'intérieur de ladite lumière (52) est en contact avec elle, accélère la vitesse du chyme.

3. Contrôleur de flux anti-obésité selon la revendication 2, dans lequel ladite structure tubulaire (40) comprend un stent tubulaire ayant une surface interne,
ladite structure de revêtement (57) étant tubulaire et fixée à ladite surface interne dudit stent en relation coaxiale avec elle.

4. Contrôleur de flux anti-obésité selon la revendication 2, dans lequel ladite surface interne (60) de ladite structure de revêtement (57) comprend un matériau qui est lubrifiant de telle sorte que, lorsque le chyme qui s'écoule à l'intérieur dudit lumen est en contact avec ledit matériau, la vitesse du chyme augmente.

5. Contrôleur de flux anti-obésité selon la revendication 2, dans lequel ladite surface interne (60) de ladite structure de revêtement (57) comprend un ePTFE qui, lorsque le chyme qui s'écoule à l'intérieur de ladite lumière est en contact avec lui, entraîne une augmentation de la vitesse du chyme.

6. Contrôleur de flux anti-obésité selon la revendication 2, dans lequel ladite surface interne (60) de ladite structure de revêtement (57) comprend un matériau qui est hydrophile de telle sorte que, lorsque le chyme qui s'écoule à l'intérieur de ladite lumière est en contact avec ledit matériau, la vitesse du chyme augmente.

7. Contrôleur de flux anti-obésité selon la revendication 1, dans lequel ladite structure de contrôle (55a) comprend une structure de restriction (65) fixée à ladite structure interne (45a) de ladite structure tubulaire (40a), ladite structure de restriction (65) constituant un obstacle à l'écoulement du chyme à l'intérieur de ladite lumière (52a), entraînant une réduction de la vitesse du chyme.

8. Contrôleur de flux anti-obésité selon la revendication 7, dans lequel ladite structure de restriction (65) comprend une structure de déflexion (67) fixée à ladite surface interne (45a) de ladite structure tubulaire (40a), ladite structure de déflexion (67) procurant un obstacle à l'écoulement du chyme à l'intérieur de ladite lumière (52a), entraînant une réduction de la vitesse du chyme.
